# FASCICULE DE BREVET EUROPEEN

(11) **EP 2 229 111 B1**
(45) Date de publication et mention de la délivrance du brevet: **14.10.2015**
(21) Numéro de dépôt: 08872533.8
(22) Date de dépôt: 12.12.2008
(51) Int. Cl.: A61B 17/86, A61B 17/60, A61B 17/80

(54) **DOUILLE DE BLOCAGE POUR UN DISPOSITIF D'OSTEOSYNTHESE ET DISPOSITIF D'OSTEOSYNTHESE COMPRENANT UNE TELLE DOUILLE**
BLOCKIERUNGSRING FÜR EINE OSTEOSYNTHESEVORRICHTUNG UND OSTEOSYNTHESEVORRICHTUNG MIT EINEM SOLCHEN RING
BLOCKING RING FOR OSTEOSYNTHESIS DEVICE AND OSTEOSYNHESIS DEVICE INCLUDING SUCH RING

(30) Priorité: 13.12.2007 FR 0708686
(43) Date de publication de la demande: 22.09.2010
(73) Titulaire: Worcel, Alexandre, Parc de Mongarny 95680 Montlignon (FR); Worcel, Manuel, 75012 Paris (FR); Worcel, Julia, 95680 Montlignon (FR); Worcel, Marie, 95680 Montlignon (FR); Paoli, Albert, 95300 Pontoise (FR); Paoli, Myriam, 95300 Pontoise (FR); Paoli, Edouard, 95300 Pontoise (FR); Paoli, Jean, 95300 Pontoise (FR); Lombardo-Fiault, Bernard, 75011 Paris (FR); Laumonier, Alain, 95690 Nesles La Vallée (FR); Laumonier, Rémi, 95300 Pontoise (FR); Laumonier, Bruno, 95690 Nesles La Vallée (FR); Laumonier, Yves, 95690 Nesles La Vallée (FR); Laumonier, Nicolas, 95300 Pontoise (FR); Ateliers Laumonier, SAS, 95690 Nesles La Vallée (FR)
(72) Inventeur: WORCEL, Alexandre, 95680 Montlignon (FR)
(74) Mandataire: Gendron, Vincent Christian
(86) Numéro de dépôt international: PCT/FR2008/001732
(87) Numéro de publication internationale: WO 2009/103886

(56) Documents cités:
- WO-A-98/01079
- WO-A-2004/069067
- WO-A-2008/029032
- DE-U1- 29 913 994
- US-A- 5 904 683
- US-A1- 2007 010 817

## Description

La présente invention concerne une douille de blocage pour une broche de maintien destinée à être vissée dans une partie osseuse. Elle concerne également un ensemble comprenant une telle douille et une bague destinée à être insérée dans un orifice d'une plaque de maintien. Elle concerne enfin un dispositif de d'ostéosynthèse.

Un dispositif d'ostéosynthèse comprend une plaque de maintien, au moins deux broches destinées à être vissées dans deux parties osseuses, et des douilles de blocage pour assurer une fixation des broches et ainsi solidariser la plaque de maintien avec les deux parties osseuses.

Un problème important dans ce domaine technique est de concevoir un dispositif d'ostéosynthèse offrant, à la fois, un blocage efficace des broches, pour éviter qu'elles ne se dévissent sous l'effet de contraintes mécaniques ou de vibrations, et un dévissage aisé de la broche lorsque le praticien souhaite enlever le dispositif d'ostéosynthèse.

EP-1 583 478 divulgue un dispositif d'ostéosynthèse dans lequel les axes de la broche et de la douille de blocage sont décalés angulairement. Dans ce dispositif d'ostéosynthèse, les trous de la plaque de maintien sont filetés et reçoivent une douille intermédiaire qui présente un alésage fileté pour recevoir une broche et une douille de blocage. WO 2004/069067 A divulgue les caractéristiques du préambule de la revendication 1.

Pour la mise en place du dispositif d'ostéosynthèse, le praticien, après avoir percé la plaque de maintien en au moins deux endroits choisis pour assurer sa fixation sur la partie osseuse, effectue un perçage de cette partie osseuse en guidant un forêt perpendiculairement à la plaque de maintien. Ensuite, il met en place la douille intermédiaire et visse la broche filetée dans la partie osseuse à la longueur souhaitée. Il introduit alors la douille de blocage sur la broche et procède au vissage de cette douille de blocage dans la douille intermédiaire.

On comprend qu'en raison du décalage angulaire existant entre les axes de la broche et de la douille de blocage on réalise au cours de ce vissage un coincement progressif de la douille de blocage dans la douille intermédiaire. On obtient ainsi un blocage irréversible de la broche par rapport à la plaque de maintien.

Ce dispositif est efficace mais il présente cependant plusieurs inconvénients.

Tout d'abord, la tolérance est faible sur l'écart angulaire entre les axes de la broche et de la douille de blocage. La fabrication de la douille intermédiaire est donc délicate, ce qui induit un coût de fabrication élevé.

Par ailleurs, cet écart angulaire, s'il permet d'assurer un blocage efficace, présente le désavantage que ce blocage se produit déjà dès le début du vissage de la douille de blocage. Il peut donc arriver que la douille de blocage ne pénètre qu'insuffisamment dans la plaque de maintien.

Enfin, dans le cas d'une plaque de faible épaisseur (par exemple inférieure à 3 mm), la douille intermédiaire et la douille de blocage doivent être insérées partiellement dans la partie osseuse pour permettre un bon verrouillage du dispositif. Ceci ne permet pas d'utiliser ce dispositif de l'art antérieur pour les os supérieurs (bras, avant-bras, mains, poignet, visage) qui sont trop fins.

L'invention a pour but de s'affranchir des limitations des dispositifs d'ostéosynthèse de l'art antérieur et est définie par les caractéristiques de la revendication 1.

La présente invention atteint son but en proposant une douille de blocage pour une broche de maintien filetée destinée à être vissée dans une pièce osseuse, ladite douille comportant une première partie ayant un alésage axial pour recevoir ladite broche de maintien, la périphérie de ladite première partie étant filetée pour coopérer avec une plaque de maintien, ladite douille étant caractérisée en ce qu'elle comprend en outre, dans le prolongement de ladite première partie, une deuxième partie de plus faible épaisseur que la première partie et présentant un alésage axial identique, cette deuxième partie étant déformable et ainsi apte à bloquer, par déformation, ladite douille sur ladite broche.

Selon un mode de réalisation préféré, l'alésage de la première partie de la douille est fileté.

De manière avantageuse, la première partie de la douille a une forme extérieure cylindrique ou tronconique.

L'invention a également pour objet un ensemble comprenant une douille selon l'invention et une bague, ladite bague étant destinée à être insérée dans un orifice d'une plaque de maintien, la bague ayant un alésage tronconique filetée correspondant au filetage de la douille et une fente radiale.

De manière avantageuse, la bague comporte une fente radiale.

Selon un mode de réalisation particulier, la bague présente une périphérie convexe, destinée à coopérer avec un orifice concave d'une plaque de maintien.

L'invention a enfin pour objet un dispositif d'ostéosynthèse comprenant une plaque de maintien ayant au moins deux orifices, des broches destinées à être insérées dans lesdits orifices pour être vissées dans une pièce osseuse, et des douilles de blocage ou des ensembles selon l'invention.

D'autres particularités et avantages de l'invention ressortiront à la lecture de la description faite ci-après d'un mode de réalisation particulier de l'invention, donné à titre indicatif mais non limitatif, en référence aux dessins annexés sur lesquels :
- la figure 1 représente schématiquement un dispositif d'ostéosynthèse,
- la figure 2 représente en coupe longitudinale une partie d'un dispositif d'ostéosynthèse montée sur une partie osseuse comprenant une douille selon un premier mode de réalisation de l'invention,
- la figure 3 représente le dispositif de la figure 1 après cisaillage de la douille de blocage et de la broche,
- la figure 4 représente en coupe longitudinale une partie d'un dispositif d'ostéosynthèse montée sur une partie osseuse comprenant une douille selon un deuxième mode de réalisation de l'invention, et
- la figure 5 représente une vue selon la direction D du dispositif représenté sur la Figure 4.

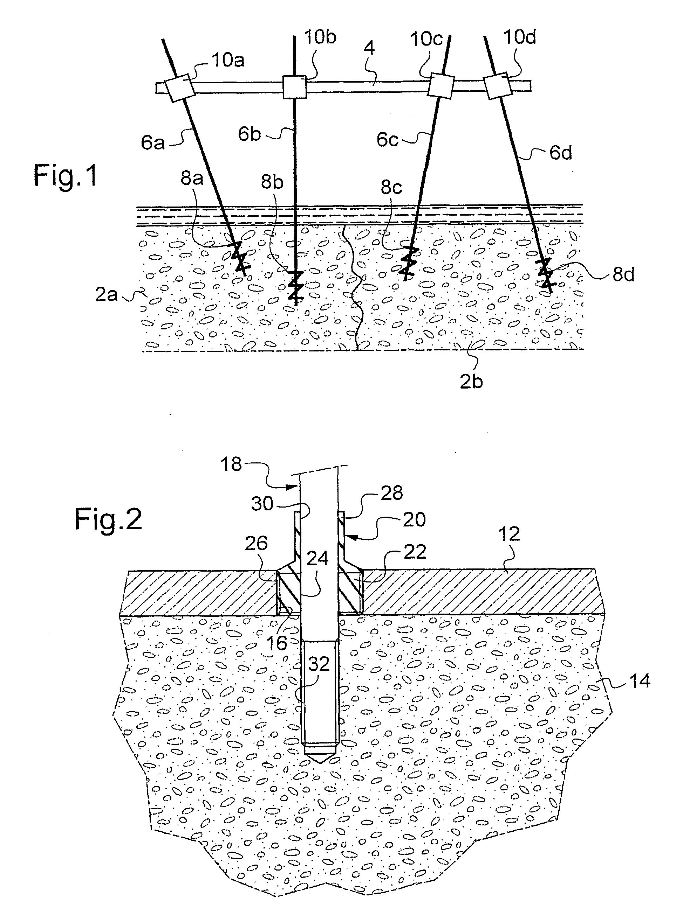

On a représenté schématiquement sur la figure 1 un dispositif d'ostéosynthèse pour solidariser deux parties osseuses 2a, 2b à une plaque de maintien 4. La plaque de maintien 4 comporte des orifices pour le passage de broches 6a, 6b, 6c, 6d dont une partie au moins comporte un filetage 8a, 8b, 8c, 8d pour le vissage de la broche dans l'une des parties osseuses. Le dispositif d'ostéosynthèse comporte deux broches par partie osseuse, mais on comprend qu'il pourrait ne comprendre qu'une seule broche par partie osseuse dans certains cas. On note que les broches peuvent être vissées selon une direction perpendiculaire à celle du plan de la plaque de maintien 4. C'est le cas de la broche 8b. Elles peuvent également être vissées selon une direction inclinée par rapport à cette direction perpendiculaire. C'est le cas des broches 8a, 8c, 8d.

Des douilles de blocage 10a, 10b, 10c, 10d sont prévues dans les orifices de la plaque de maintien 4 pour solidariser les broches avec cette plaque de maintien.

Des modes de réalisation d'une douille de blocage selon l'invention vont maintenant être décrites en référence aux figures 2 à 5.

La figure 2 illustre une partie d'un dispositif d'ostéosynthèse comprenant une douille selon un premier mode de réalisation de l'invention. On a représenté sur cette figure une seule broche, mais on comprend qu'un dispositif d'ostéosynthèse comporte au moins deux broches pour solidariser deux parties osseuses.

Sur la figure 2, une plaque de maintien 12 est placée au-dessus d'une partie osseuse 14. La plaque de maintien comprend un orifice 16 pour recevoir une broche 18 et une douille de blocage 20.

Selon l'invention la douille de blocage 20 comprend une première partie 22 ayant un alésage axial 24 pour le passage de la broche 18 cet alésage pouvant être ou non fileté, la périphérie de la première partie 22 de la douille de blocage comportant un filetage 26 pour être vissée dans l'orifice de la plaque de maintien, et une deuxième partie 28, dans le prolongement de la première partie 22, de plus faible épaisseur que la première partie et présentant un alésage axial 30 identique a celui de la première partie.

L'épaisseur de la deuxième partie 28 est choisie en fonction du matériau dont est constituée la douille 20, de manière que, lors du cisaillement de la broche et de la deuxième partie, la partie restante de la deuxième partie se déforme et s'écrase contre la partie supérieure de la broche restante, assurant ainsi un blocage de celle-ci par rapport à la plaque de maintien.

La mise en place du dispositif d'ostéosynthèse s'effectue de la manière suivante. Le praticien perce la plaque de maintien en au moins deux endroits choisis pour assurer sa fixation sur les parties osseuses. Il effectue ensuite un perçage de chaque partie osseuse en guidant un foret perpendiculairement à la plaque de maintien. Il prépositionne ensuite la douille de blocage 20 en la vissant partiellement dans l'orifice 16. Il peut également visser complètement la douille de blocage 20 dans l'orifice 16, notamment si l'alésage axial de la première partie 22 de la douille de blocage n'est pas fileté. La broche 18, dont la partie inférieure comporte un filetage 32 est ensuite vissée dans la partie osseuse à la longueur souhaitée, la douille de blocage 20, ou à tout le moins sa deuxième partie, ayant alors une fonction secondaire de visée pour guider la broche lors de son vissage. Si la douille de blocage 20 n'a été que prépositionnée, elle est maintenant vissée complètement dans l'orifice 16.

Le praticien cisaille alors la broche 18 et la douille 20 sensiblement au niveau de la base de la deuxième partie 28, c'est-à-dire au niveau de la jonction entre la première partie 22 et de la deuxième partie 28. Un repère, tel qu'une légère rainure, peut être prévu à la périphérie de la douille pour définir de manière précise la position de l'outil de cisaillement.

La partie restante 34 de la deuxième partie 28 de la douille de blocage 22 est déformée vers la broche 18 par l'action de cisaillement. Un blocage de la broche 18 par rapport à la douille de blocage 22, et donc par rapport à la plaque de maintien 12, est ainsi obtenu par sertissage.

Ainsi, lorsque le praticien procède au cisaillage de la broche 18 et la douille 20 au niveau de la jonction entre la première partie 22 et la deuxième partie 28, au moyen d'une pince adaptée, les mors de la pince tendent d'abord à enserrer la douille de blocage 20 puis en se rejoignant, la déforme pour finalement sectionner à la fois la douille de blocage 20 et la broche 18. Ainsi apparaît un bord libre cisaillé de la deuxième partie formant une partie restante 34, solidaire de la première partie. Deux portions diamétralement opposées dudit bord libre sont alors rapprochées l'une de l'autre sous l'effet du cisaillement et recouvrent sensiblement l'extrémité libre cisaillée de la broche 18. La partie restante 34 de la deuxième partie 28 de la douille de blocage 22 ainsi déformée vers la broche 18 en l'enserrant, permet d'obtenir un blocage de la broche 18 par rapport à la douille de blocage 22 par sertissage.

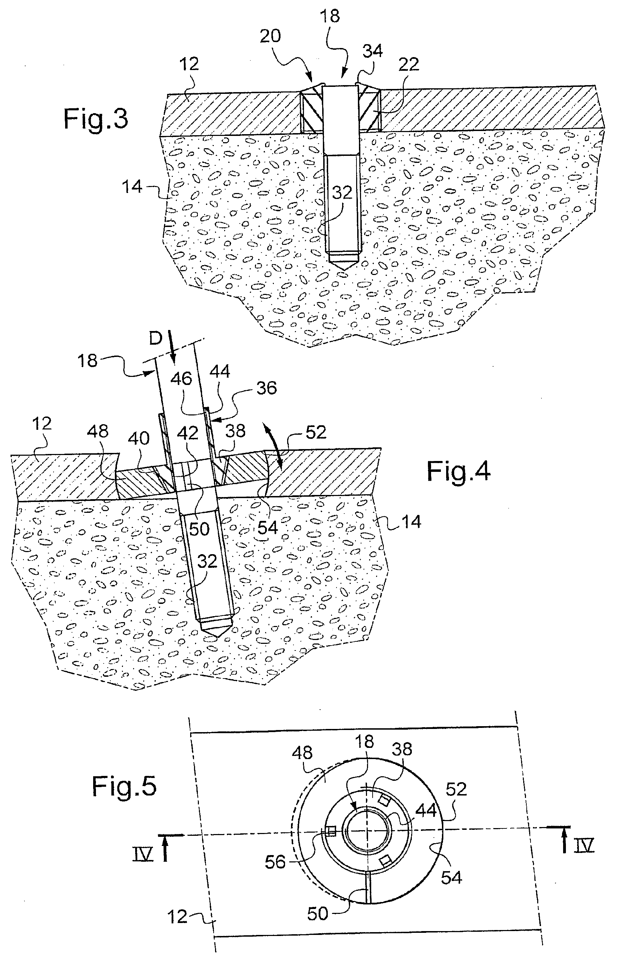

On se trouve alors dans la situation de la figure 3, sur laquelle les éléments identiques à ceux de la figure 2 portent les mêmes références.

La douille de blocage selon l'invention est particulièrement intéressante en ce qu'elle offre une excellente efficacité de blocage pour des broches de petit diamètre, tel que par exemple un diamètre de 0,8 à 1,8 mm, qui correspond au type de broche utilisée pour des parties osseuses du poignet, de la main ou du visage.

Ainsi, on a constaté expérimentalement une résistance en traction de plus de 200 KG sur un banc d'essai ne pouvant pas mesurer plus de 200 kg pour une broche de 4 mm de diamètre et une douille en acier chirurgical 316L dont la deuxième partie a une épaisseur de 1 mm. Dans cette essai, la douille n'a pas de filetage interne. On comprend que la résistance en traction serait grandement augmentée dans le cas d'une douille à filetage interne. Ceci permettrait notamment d'obtenir une excellente résistance en traction, même avec des broches de petits diamètres.

Bien entendu, la douille de blocage selon l'invention est également utilisable et efficace avec des broches de diamètre plus important.

On a représenté sur la figure 4, en coupe, une partie d'un dispositif d'ostéosynthèse comportant une douille de blocage selon un deuxième mode de réalisation de l'invention, et sur la figure 5 une vue de dessus selon une direction D parallèle à celle de l'axe de la broche.

La douille de blocage 36 comprend une première partie 38 de forme tronconique filetée (filetage 40) pourvue d'un alésage axial 42 et une deuxième partie 44, dans le prolongement de la première partie, et comprenant un alésage axial 46 identique à celui de la première partie 38. Selon l'invention, l'épaisseur de la deuxième partie est choisie en fonction du matériau utilisé pour que cette deuxième partie soit déformable et bloque ainsi la broche, par déformation, lors du cisaillement celle-ci.

Le deuxième mode de réalisation de l'invention diffère essentiellement du premier mode de réalisation en ce que la douille de blocage est montée sur une bague 48 orientable dans l'orifice 50 de la plaque de maintien. A cette fin, la bague présente un bord convexe 52 et l'orifice de la plaque de maintien 12 un bord concave correspondant 54.

La mise en place de la broche se fait de la même manière que dans le cas du dispositif décrit en référence aux figures 2 et 3. Le praticien perce tout d'abord la plaque en au moins deux endroits. Il forme ensuite le bord concave de l'orifice pour permettre l'insertion ultérieure de la bague. Il perce alors la partie osseuse selon l'inclinaison choisie au moyen d'un foret. La bague 48 est ensuite mise en place dans l'orifice de la plaque de maintien, cette insertion étant facilitée par la fente radiale 50 de la bague 48. La douille de blocage 36 est vissée au moins partiellement sur la bague 48 pour bloquer son orientation et guider ainsi la broche 18. Lorsque la broche a été vissée de la longueur souhaitée, et que la douille de blocage a été complètement vissée sur la bague 48, le praticien cisaille la douille de blocage 36 et la broche 18 au niveau de la partie inférieure de la deuxième partie 44 de la douille de blocage 36, déformant la partie résiduelle de cette deuxième partie et bloquant ainsi la broche 18.

De manière classique, la douille de blocage suivant chacun des modes de réalisation décrits comporte une ou plusieurs encoches, telle que les encoches 56 visibles sur la figure 5, pour permettre au praticien, à l'aide d'un tournevis adapté, de dévisser l'ensemble broche/douille de blocage lorsqu'il souhaite retirer la plaque de maintien du patient.

## Revendications

1. Douille de blocage (20, 36) pour une broche (18) de maintien filetée destinée à être vissée dans une pièce osseuse (14), ladite douille comportant une première partie (22, 38) ayant un alésage axial (24, 42) pour recevoir ladite broche de maintien, la périphérie de ladite première partie étant filetée pour coopérer avec une plaque de maintien, ladite douille comprenant en outre, dans le prolongement de ladite première partie, une deuxième partie (28, 44) déformable de plus faible épaisseur que la première partie, **caractérisée en ce que** la première partie et la deuxième partie présentent un alésage axial (30, 46) identique, et **en ce que** l'épaisseur de la deuxième partie est choisie en fonction du matériau dont est constituée la douille de manière que le cisaillage de la broche (18) et la douille (20) au niveau de la jonction entre la première partie (22) et la deuxième partie (28) déforme la partie restante (34) de la deuxième partie (28) de la douille de blocage (22) vers la broche (18) de manière à obtenir un blocage de la broche (18) par rapport à la douille de blocage (22) par sertissage.

2. Douille selon la revendication 1, **caractérisée en ce que** la l'alésage (24) de la première partie (22) de la douille comprend un filetage (26).

3. Douille selon l'une des revendications 1 ou 2, **caractérisée en ce que** la première partie (22) a une forme extérieure cylindrique.

4. Douille selon la revendication 1, **caractérisée en ce que** la première partie (38) a une forme extérieure tronconique.

5. Ensemble comprenant une douille selon la revendication 4 et une bague (48), ladite bague étant destinée à être insérée dans un orifice d'une plaque de maintien (12), la bague ayant un alésage tronconique filetée correspondant au filetage de la douille et une fente radiale.

6. Ensemble selon la revendication 5, **caractérisé en ce que** la bague (48) comporte une fente radiale (50).

7. Ensemble selon la revendication 6, **caractérisé en ce que** la bague présente une périphérie convexe (52), destinée à coopérer avec un orifice concave (54) d'une plaque de maintien.

8. Dispositif d'ostéosynthèse comprenant une plaque de maintien (12) ayant au moins deux orifices, des broches (18) destinées à être insérées dans lesdits orifices pour être vissées dans une pièce osseuse (14) et des douilles de blocage (20, 36) selon l'une des revendications 1 à 3.

9. Dispositif d'ostéosynthèse comprenant une plaque de maintien (12) ayant au moins deux orifices, des broches (18) destinées à être insérées dans lesdits orifices pour être vissées dans une pièce osseuse et des ensembles selon l'une des revendications 5 à 7.

## Patentansprüche

1. Blockierungshülse (20, 36) für einen mit Gewinde versehenen Haltedorn (18), der in ein Knochenstück (14) geschraubt werden soll, wobei die Hülse einen ersten Abschnitt (22, 38) mit einer axialen Bohrung (24, 42) zum Aufnehmen des Haltedorns umfasst, wobei der Umfang des ersten Abschnitts mit einem Gewinde versehen ist, um mit einer Halteplatte zusammenzuwirken, wobei die Hülse außerdem in der Verlängerung des ersten Abschnitts einen verformbaren zweiten Abschnitt (28, 44) mit einer geringeren Dicke als der erste Abschnitt umfasst, **dadurch gekennzeichnet, dass** der erste Abschnitt und der zweite Abschnitt eine identische axiale Bohrung (30, 46) aufweisen und dass die Dicke des zweiten Abschnitts in Abhängigkeit von dem Material ausgewählt ist, aus dem die Hülse gebildet ist, so dass das Abschneiden des Dorns (18) und der Hülse (20) auf der Höhe der Verbindung zwischen dem ersten Abschnitt (22) und dem zweiten Abschnitt (28) den restlichen Abschnitt (34) des zweiten Abschnitts (28) der Blockierungshülse (22) zum Dorn (18) hin verformt, um eine Blockierung des Dorns (18) in Bezug auf die Blockierungshülse (22) durch Einfassen zu erhalten.

2. Hülse nach Anspruch 1, **dadurch gekennzeichnet, dass** die Bohrung (24) des ersten Abschnitts (22) der Hülse ein Gewinde (26) umfasst.

3. Hülse nach einem der Ansprüche 1 oder 2, **dadurch gekennzeichnet, dass** der erste Abschnitt (22) eine zylindrische äußere Form aufweist.

4. Hülse nach Anspruch 1, **dadurch gekennzeichnet, dass** der erste Abschnitt (38) eine kegelstumpfförmige äußere Form aufweist.

5. Einheit mit einer Hülse nach Anspruch 4 und einem Ring (48), wobei der Ring in eine Öffnung einer Halteplatte (12) eingesetzt werden soll, wobei der Ring eine kegelstumpfförmige Gewindebohrung, die dem Gewinde der Hülse entspricht, und einen radialen Schlitz aufweist.

6. Einheit nach Anspruch 5, **dadurch gekennzeichnet, dass** der Ring (48) einen radialen Schlitz (50) umfasst.

7. Einheit nach Anspruch 6, **dadurch gekennzeichnet, dass** der Ring einen konvexen Umfang (52) aufweist, der mit einer konkaven Öffnung (54) einer Halteplatte zusammenwirken soll.

8. Osteosynthesevorrichtung mit einer Halteplatte (12) mit mindestens zwei Öffnungen, Dornen (18), die in die Öffnungen eingesetzt werden sollen, um in ein Knochenstück (14) geschraubt zu werden, und Blockierungshülsen (20, 36) nach einem der Ansprüche 1 bis 3.

9. Osteosynthesevorrichtung mit einer Halteplatte (12) mit mindestens zwei Öffnungen, Dornen (18), die in die Öffnungen eingesetzt werden sollen, um in ein Knochenstück geschraubt zu werden, und Einheiten nach einem der Ansprüche 5 bis 7.

## Claims

1. A lock ring (20, 36) for a threaded retaining pin (18) designed to be screwed into a bone piece (14), said ring comprising a first part (22, 38) having an axial bore (24, 42) to receive said fixing pin, the periphery of said first part being threaded to engage with a fixing plate; said ring further comprising, continuing on from said first part, a deformable second part (28, 44) which is thinner than the first part, **characterised in that** the first part and the second part have an identical axial bore (30, 46), and **in that** the thickness of the second part is selected as a function of the material from which the ring is formed such that the shearing of the pin (18) and of the ring (20) at the junction between the first part (22) and the second part (28) deforms the remaining part (34) of the second part (28) of the lock ring (22) towards the pin (18) in such a way as to lock the pin (18) with respect to the lock ring (22) by a crimping action.

2. The ring as claimed in claim 1, **characterised in that** the bore (24) of the first part (22) of the ring comprises a thread (26).

3. The ring as claimed in any one of claims 1 or 2, **characterised in that** the first part (22) has a cylindrical external form.

4. The ring as claimed in claim 1, **characterised in that** the first part (38) has a frustoconical external form.

5. An assembly comprising a ring as claimed in claim 4 and a collar (48), said collar being designed to be inserted into a hole in a fixing plate (12), the collar having a threaded frustoconical bore corresponding to the thread on the ring, and a radial slot.

6. The assembly as claimed in claim 5, **characterised in that** the collar (48) comprises a radial slot (50).

7. The assembly as claimed in claim 6, **characterised in that** the collar has a convex periphery (52) designed to engage with a concave hole (54) in a fixing plate.

8. An osteosynthesis device comprising a fixing plate (12) having at least two holes, pins (18) designed to be inserted into said holes for screwing into a bone piece (14), and lock rings (20, 36) as claimed in any one of claims 1 to 3.

9. An osteosynthesis device comprising a fixing plate (12) having at least two holes, pins (18) designed to be inserted into said holes for screwing into a bone piece and assemblies as claimed in any one of claims 5 to 7.
